# EUROPÄISCHE PATENTANMELDUNG

(11) **EP 0 685 464 A1**
(43) Veröffentlichungstag der Anmeldung: **06.12.1995**
(21) Anmeldenummer: 95810342.6
(22) Anmeldetag: 23.05.1995
(51) Int. Cl.: C07D 209/48, C07C 317/28, C09B 62/51, C09B 62/513

(54) **Zwischenprodukte für Reaktivfarbstoffe**

(30) Priorität: 30.05.1994 CH 1678/94
(71) Anmelder: CIBA-GEIGY AG, CH-4002 Basel (CH)
(72) Erfinder: Herzig, Paul, CH-4057 Basel (CH); Andreoli, Anton, CH-4125 Riehen (CH)

(57) **Zusammenfassung**

Verbindungen der Formel 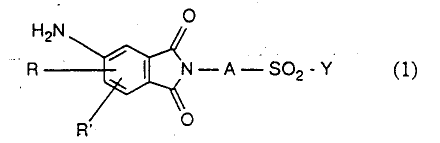
worin
R und R' unabhängig voneinander je für Wasserstoff, Sulfo, Hydroxy, C₁-C₄-Alkoxy, Halogen oder Cyano stehen,
A unsubstituiertes oder durch Halogen, Hydroxy, Sulfato, Carboxy, Cyano,
C₂-C₄-Alkanoyloxy, C₁-C₄-Alkoxycarbonyl oder Carbamoyl substituiertes oder durch eine Gruppe-O-oder-NR₁-unterbrochenes C₁-C₆-Alkylen oder unsubstituiertes oder im Phenylenteil durch C₁-C₄-Alkyl, C₁-C₄-Alkoxy, Halogen oder Sulfo substituiertes C₁-C₆-Alkylenphenylen ist,
R₁ für Wasserstoff oder C₁-C₄-Alkyl steht,
Y Vinyl oder einen Rest-CH₂-CH₂-U bedeutet, und
U eine alkalisch abspaltbare Abgangsgruppe ist, sind als Diazokomponenten für die Synthese von faserreaktiven Farbstoffen geeignet.

## Beschreibung

Die vorliegende Erfindung betrifft neue Zwischenprodukte für die Herstellung von Reaktivfarbstoffen sowie Verfahren zu ihrer Herstellung.

Faserreaktive Farbstoffe, Verfahren zu ihrer Herstellung und geeignete Zwischenprodukte für ihre Synthese sind in grosser Zahl bekannt. Angesichts der immer höheren Anforderungen an Reaktivfärbungen in bezug auf Wirtschaftlichkeit, Applikationstechnik und Echtheitsniveau ist der erreichte technische Stand aber vielfach noch nicht voll befriedigend. Die Synthese neuer Reaktivfarbstoffe ist jedoch zu einem beträchtlichen Teil an das Auffinden von neuen geeigneten Zwischenprodukten gebunden. Es besteht daher ein Bedarf nach neuen Zwischenprodukten, welche als Ausgangspunkt für die Synthese von neuen Reaktivfarbstoffen mit verbesserten Eigenschaften dienen können.

Es wurden nun überraschend neue Phthalimidverbindungen gefunden, die wertvolle Zwischenprodukte für die Synthese von Reaktivfarbstoffen darstellen.

Gegenstand der vorliegenden Erfindung sind Verbindungen der Formel 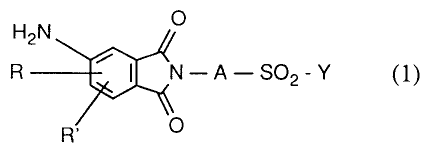
worin
R und R' unabhängig voneinander je für Wasserstoff, Sulfo, Hydroxy, C₁-C₄-Alkoxy, Halogen oder Cyano stehen,
A unsubstituiertes oder durch Halogen, Hydroxy, Sulfato, Carboxy, Cyano,
C₂-C₄-Alkanoyloxy, C₁-C₄-Alkoxycarbonyl oder Carbamoyl substituiertes oder durch eine Gruppe -O- oder -NR₁- unterbrochenes C₁-C₆-Alkylen oder unsubstituiertes oder im Phenylenteil durch C₁-C₄-Alkyl, C₁-C₄-Alkoxy, Halogen oder Sulfo substituiertes C₁-C₆-Alkylenphenylen ist,
R₁ für Wasserstoff oder C₁-C₄-Alkyl steht,
Y Vinyl oder einen Rest -CH₂-CH₂-U bedeutet, und
U eine alkalisch abspaltbare Abgangsgruppe ist.

Der Begriff Sulfo umfasst hierbei generell sowohl die freie Säure -SO₃H als auch eine beliebige Salzform, z.B. ein Alkali-, Erdalkali- oder Ammoniumsalz oder das Salz eines organischen Amins wie etwa das Natrium-, Kalium-, Lithium- oder Ammoniumsalz oder das Salz des Triethanolamins.

C₁-C₄-Alkyl bedeutet generell Methyl, Ethyl, n- oder iso-Propyl oder n-, iso-, sec.- oder tert.-Butyl. C₁-C₄-Alkoxy bedeutet generell Methoxy, Ethoxy, n- oder iso-Propoxy oder n-, iso-, sec.- oder tert.-Butoxy und vorzugsweise Methoxy oder Ethoxy. Halogen steht generell z.B. für Brom oder Chlor. Beispiele für C₂-C₄-Alkanoyloxy sind Acetyl oder Propionyl, Beispiele für C₁-C₄-Alkoxycarbonyl sind Methoxycarbonyl oder Ethoxycarbonyl. Unter C₁-C₆-Alkylen ist generell geradkettiges oder in beliebiger Weise verzweigtes C₁-C₆-Alkylen zu verstehen; Beispiele sind Methylen, 1,1- oder 1,2-Ethylen, 1,2- oder 1,3-Propylen, 1,2-, 1,3-, 1,4- oder 2,3-Butylen, 1,4-, 2,3- oder 2,4-Pentylen, 2-Methyl-1,5-pentylen und 1,6-Hexylen, wobei diese Reste wie angegeben substituiert oder mit Ausnahme von Methylen durch ein Heteroatom -O- oder -NR₁- unterbrochen sein können.

R und R' bedeuten unabhängig voneinander je bevorzugt Wasserstoff, Sulfo, Chlor, Brom, Hydroxy, Methoxy oder Cyano; besonders bevorzugt steht R für Wasserstoff, Sulfo, Chlor, Brom oder Cyano und R' für Wasserstoff; insbesondere bevorzugt bedeutet R Wasserstoff oder Sulfo und R' Wasserstoff. Eine besonders bevorzugte Ausführungsform der Erfindung betrifft Verbindungen der Formel (1), worin R und R' je Wasserstoff sind.

Bedeutet A einen C₁-C₆-Alkylenphenylenrest, handelt es sich bevorzugt um unsubstituiertes oder im Phenylenteil durch Methyl, Methoxy, Chlor oder Sulfo substituiertes Methylenphenylen oder Ethylenphenylen und besonders bevorzugt um Methylenphenylen.

A bedeutet vorzugsweise einen unsubstituierten C₁-C₆-Alkylenrest oder einen Rest der Formel

-(CH₂)ₚ-O-(CH₂)_{q}-

oder

-(CH₂)ₚ-NH-(CH₂)_{q}-,

worin p und q unabhängig voneinander je eine ganze Zahl von 1 bis 6 darstellen.

A steht besonders bevorzugt für einen C₂-C₄-Alkylenrest oder für einen Rest der Formel -(CH₂)₂₋₄-O-(CH₂)₂₋₄- oder -(CH₂)₂₋₄-NH-(CH₂)₂₋₄-.

Insbesonders bevorzugte Bedeutungen von A sind 1,2-Ethylen, 1,3-Propylen, 1,4-Butylen oder ein Rest der Formel

-(CH₂)₂₋₃-O-(CH₂)₂₋₃-

oder

-(CH₂)₂₋₃-NH-(CH₂)₂₋₃-.

Eine besonders bevorzugte Ausführungsform der vorliegenden Erfindung betrifft Verbindungen der Formel (1), worin A 1,2-Ethylen, 1,3-Propylen oder einen Rest der Formel -(CH₂)₂-O-(CH₂)₂- bedeutet.

Geeignete alkalisch abspaltbare Abgangsgruppen U sind z.B. Halogen wie etwa Chlor oder Brom, Acyloxy wie etwa Acetoxy oder Benzoyloxy, Phosphato, Sulfato oder Thiosulfato.

Beispiele für geeignete Reste Y sind dementsprechend Vinyl, β-Brom- oder β-Chlorethyl, β-Acetoxyethyl, β-Benzoyloxyethyl, β-Phosphatoethyl, β-Sulfatoethyl und β-Thiosulfatoethyl. Y steht bevorzugt für Vinyl oder β-Sulfatoethyl.

Von besonderem Interesse sind Verbindungen der zuvor angegebenen Formel (1), worin R Wasserstoff, Sulfo, Chlor, Brom oder Cyano; R' Wasserstoff; A unsubstituiertes C₁-C₆-Alkylen oder einen Rest der Formel

-(CH₂)ₚ-O-(CH₂)_{q}-

oder

-(CH₂)ₚ-NH-(CH₂)_{q}-,

worin p und q unabhängig voneinander je eine ganze Zahl von 1 bis 6 darstellen; und Y Vinyl, β-Brom- oder β-Chlorethyl, β-Acetoxyethyl, β-Benzoyloxyethyl, β-Phosphatoethyl, β-Sulfatoethyl oder β-Thiosulfatoethyl bedeuten.

Von ganz besonderem Interesse sind Verbindungen der zuvor angegebenen Formel (1), worin R Wasserstoff oder Sulfo; R' Wasserstoff; A C₂-C₄-Alkylen oder einen Rest der Formel -(CH₂)₂₋₄-O-(CH₂)₂₋₄- oder -(CH₂)₂₋₄-NH-(CH₂)₂₋₄-; und Y Vinyl oder β-Sulfatoethyl bedeuten.

Eine insbesondere bevorzugte Ausführungsform der vorliegenden Erfindung stellen Verbindungen der zuvor angegebenen Formel (1) dar, worin R und R' je Wasserstoff, A 1,2-Ethylen, 1,3-Propylen oder einen Rest der Formel -(CH₂)₂-O-(CH₂)₂- und Y β-Sulfatoethyl bedeuten.

Die Verbindungen der Formel (1) können erhalten werden z.B., indem man
a) Phthalsäureanhydrid mit einer Verbindung der Formel

   H₂N -A-S-CH₂-CH₂-U₁ (2)

   zur Verbindung der Formel 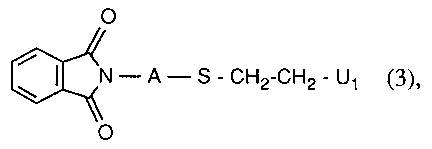 umsetzt, worin für A jeweils die zuvor angegebenen Bedeutungen und Bevorzugungen gelten und U₁ unabhängig die zuvor für U angegebene Bedeutung hat oder für Hydroxy steht,
b) die Thioetherverbindung der Formel (3) zur entsprechenden Sulfonylverbindung der Formel 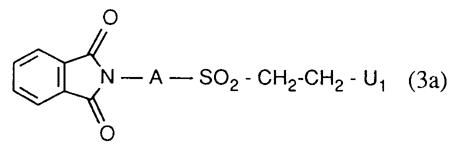 oxidiert,
c) die erhaltene Verbindung der Formel (3a) selektiv nitriert zur Verbindung der Formel 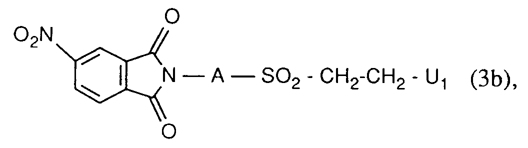
d) die erhaltene Verbindung der Formel (3b) zur Aminoverbindung der Formel 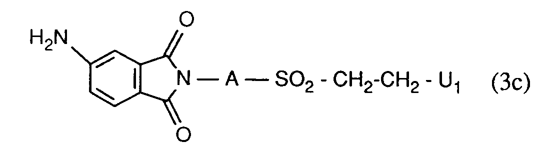 reduziert und gegebenenfalls während oder nach der Synthese der Verbindung der Formel (3a), (3b) oder (3c) einen der zuvor genannten Reste R und/oder R' in das Molekül einführt oder den Rest U₁ in einen beliebigen Rest U umwandelt.

Die Kondensation des Phthalsäureanhydrids mit dem Amin der Formel (2) im Schritt a) erfolgt z.B. in wässrigem oder wässrig-organischem Medium bei einer Temperatur von ca. 40 bis 200°C und vorzugsweise 50 bis 150°C und Normaldruck unter Abdestillieren des entstehenden Wassers.

Die Oxidation der Thioetherverbindung der Formel (3) zur Sulfonylverbindung der Formel (3a) im Schritt b) kann nach verschiedenen Methoden erfolgen, z.B. mit Wasserstoffperoxid mit oder ohne Zusatz von Wolfram- oder Vanadinverbindungen als Katalysator, ferner mit Peressigsäure, Kaliumpermanganat oder Chromsäure, oder mit Chlor/Salzsäure je in wässrigem, wässrig-organischem oder organischem Medium.

Die Nitrierung der Verbindung der Formel (3a) zur Verbindung der Formel (3b) im Schritt c) findet vorteilhaft in einem Gemisch von konz. Schwefelsäure und konz. Salpetersäure bei Raumtemperatur oder etwas darüber, z.B. bei einer Temperatur von 10 bis 100°C oder vorzugsweise 30°C bis 50°C, statt.

Die Reduktion der Nitroverbindung der Formel (3b) zur Aminoverbindung der Formel (3c) im Schritt d) erfolgt z.B. durch katalytische Hydrierung mit Pd/Kohlenstoff in wässrigem, wässrig-organischem oder organischem Medium bei Raumtemperatur bis etwa 60°C; enthält das zur Hydrierung verwendete Medium organische Lösungsmittel, so kommen hierbei in erster Linie Alkohole wie Ethanol sowie Essigester oder Tetrahydrofuran in Frage. Das Hydriergemisch kann auch einen Puffer enthalten, z.B. einen Essigsäure/Acetatpuffer. Die Reduktion kann auch mit Fe/Salzsäure oder Fe/Essigsäure in wässriger Lösung durchgeführt werden.

Die Verbindungen der Formel (3c), worin U₁ Hydroxy bedeutet, können durch Behandlung mit Sulfatierungsmitteln, Phosphorylierungsmitteln, Halogenierungsmitteln oder Alkyl- oder Arylcarbonsäurehalogeniden wie Benzoylchlorid oder Acetylchlorid in die entsprechenden Verbindungen der Formel (1), worin Y einen Rest -CH₂-CH₂-U ist, überführt werden.

Geeignete Sulfatierungsmittel sind beispielsweise konz. Schwefelsäure sowie Chlorsulfonsäure und Amidosulfonsäure oder andere SO₃ abgebenden Verbindungen. Die Sulfatierung der Hydroxygruppe U₁ zur Sulfatogruppe U kann auch im Verlauf der Synthese der Verbindungen der Formel (3c), z.B. im Nitrierungsschritt c), erfolgen. Als Halogenierungsmittel können beispielsweise Thionylchlorid oder Thionylbromid verwendet werden. Geeignete Phosphorylierungsmittel sind z.B. konz. Phosphorsäure, Pyro-, Meta- oder Polyphosphorsäure, Phosphorsäurealkylester, Phosphoroxytrichlorid oder Gemische von Phosphorsäure und Phosphor-(V)-oxid.

Wird in die Verbindung der Formel (3c) ein Rest R oder R' eingeführt, so kommt hier z.B. die Sulfonierung oder Halogenierung, d.h. die Einführung einer Sulfo- oder Halogengruppe, in Frage, die in an sich bekannter Weise, z.B. mit konz. Schwefelsäure, Oleum oder Chlorsulfonsäure bzw. mit Chlor oder Brom in Gegenwart von Lewissäuren, erfolgen kann. Die Verbindungen der Formel (1) mit R und/oder R'= Sulfo oder Halogen können wiederum als Ausgangsprodukte für die Synthese der entsprechenden Verbindungen mit R und/oder R' = Hydroxy, C₁-C₄-Alkoxy oder Cyano dienen, die nach üblichen in den Lehrbüchern der organischen Chemie beschriebenen Methoden erfolgen kann.

Die Verbindungen der Formel (1) stellen wertvolle, neue aromatische Amine dar, die in üblicher Weise, z.B. durch Einwirkung salpetriger Säure in wässrig-mineralsaurer Lösung bei tiefer Temperatur, diazotiert und anschliessend mit beliebigen Kupplungskomponenten im sauren, neutralen oder schwach alkalischen pH-Bereich gekuppelt werden können. Die dabei erhaltenen neuen Mono- oder Polyazofarbstoffe sind faserreaktiv, d.h. sie vermögen mit den Hydroxylgruppen der Cellulose oder mit den reaktiven Zentren von natürlichen und synthetischen Polyamiden unter Bildung kovalenter chemischer Bindungen zu reagieren, und ergeben auf den genannten Fasermaterialien Färbungen und Drucke mit guten Allgemeinechten, insbesondere guten Licht- und Nassechtheiten.

Ein weiterer Gegenstand der vorliegenden Erfindung betrifft Verbindungsgemische enthaltend je eine Verbindung der Formel 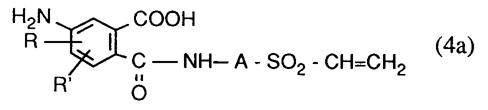
und 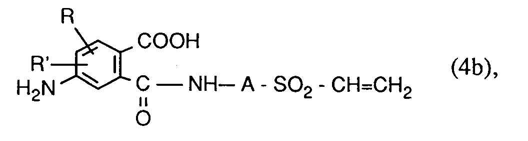
worin für A, R und R' jeweils die zuvor angegebenen Bedeutungen und Bevorzugungen gelten; die genannten Verbindungsgemische stellen ebenfalls wertvolle neue Zwischenprodukte für die Synthese von faserreaktiven Farbstoffen für Cellulose- und Polyamidfasermaterialien dar und können z.B. durch einfache Behandlung einer erfindungsgemässen Verbindung der Formel (1) in einem wässrigen alkalischen Medium erhalten werden. Das alkalische Medium weist z.B. einen pH-Wert von 8-14 und vorzugsweise 9-12 auf und kann mittels üblicher Basen, z.B. Natriumhydroxid, hergestellt werden. Beim Behandeln der Verbindungen der Formel (1) in einem solchen alkalischen Medium tritt eine Ringöffnung, wobei die Komponenten der Formel (4a) und (4b) im allgemeinen in einem stöchiometrischen Verhältnis, d.h. in etwa im Verhältnis 1:1, anfallen.

Die nachfolgenden Beispiele dienen der Erläuterung der Erfindung. Die Temperaturen sind in Celsiusgraden angegeben, Teile sind Gewichtsteile, und Prozentangaben beziehen sich auf Gew.-%, sofern nicht anders vermerkt. Gewichtsteile stehen zu Volumenteilen im Verhältnis von Kilogramm zu Liter.

### Beispiel 1:

Zu einer Lösung aus 121 Teilen 2-Amino-2'-hydroxy-diethylsulfid in 121 Teilen Wasser werden 148 Teile Phthalsäureanhydrid eingetragen. Die Temperatur lässt man dabei auf 50°C ansteigen. Nach dem Eintragen erwärmt man die entstandene Suspension auf Rückfluss und destilliert bei Normaldruck das entstehende Wasser ab. Anschliessend erhöht man die Temperatur auf 150°C und lässt eine weitere Stunde bei dieser Temperatur rühren. Man erhält eine Schmelze von N-(2-(2-Hydroxy-ethylthio)-ethyl)-phthalimid der Formel 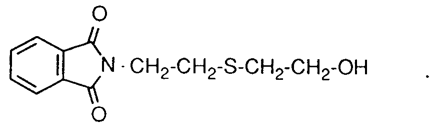
Man kühlt die entstandene Schmelze auf 80°C ab und gibt dann 8 Teile Natriumacetat und 0,5 Teile Wolfram(VI)-oxid dazu. Bei einer Temperatur von 75-80°C werden nun innert 2 Stunden 214 Teile Wasserstoffperoxid 35% zugetropft und weitere 2 Stunden bei 80°C und schwachem Peroxidüberschuss nachgerührt, bis mittels HPLC kein Sulfoxid mehr nachweisbar ist. Zur Ausfällung der entstandenen Emulsion giesst man die heisse Reaktionsmasse auf 500 Teile kaltes Wasser, filtriert und wäscht mit Wasser peroxidfrei. Nach dem Trocknen im Vakuum bei 60°C erhält man 253 Teile N-(2-(2-Hydroxy-ethylsulfonyl)-ethyl)-phthalimid der Formel 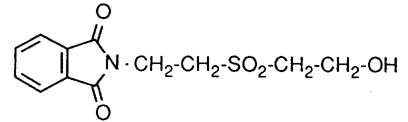
mit einem Schmelzpunkt von 120-122°C und einer Reinheit (HPLC) von 99%.
¹H-NMR-Analyse (gemessen in DMSO-d₆ mit TMS als Standard):
3,30 ppm (2H;t), 3,50 ppm (2H;t), 3,79 ppm (2H;q), 4,05 ppm (2H;t), 5,17 ppm (1H;t), 7,8 - 7,9 ppm (4H).

### Beispiel 2:

Zu 735 Teilen Schwefelsäure 100% werden unter Rühren bei 20-30°C 253 Teile N-(2-(2-Hydroxyethylsulfonyl)ethyl)phthalimid eingetragen. Man lässt eine Stunde bei Raumtemperatur nachrühren bis eine vollständige Lösung vorliegt. Nun werden bei einer Temperatur von 10-15°C 113 Teile Mischsäure 50% innert 3 Stunden zugetropft. Danach erwärmt man auf 40°C und lässt weitere 12 Stunden bei dieser Temperatur nachrühren. Zur Isolierung trägt man das Nitriergemisch bei max. 5°C auf 2500 Teile Eis aus. Die klare Lösung wird mit 250 Teilen Kaliumsulfat versetzt und eine Stunde bei 10°C verrührt. Das ausgefallene Produkt wird abfiltriert und mit Kaliumsulfatlösung 10% gewaschen. Man erhält 1195 Teile feuchtes 4-Nitro-N-(2-(2-sulfato-ethylsulfonyl)-ethyl)-phthalimid der Formel 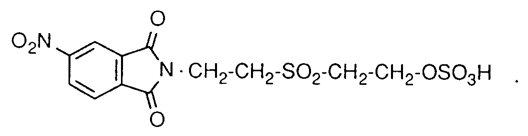
¹H-NMR-Analyse (gemessen in DMSO-d₆ mit TMS als Standard): 3,45 ppm (2H, t); 3,50 ppm (2H, t); 4,05 ppm (2H, t); 4,10 ppm (2H, t); 8,16 ppm (1H, d); 8,52 ppm (1H, s); 8,63 ppm (1H, d).

### Beispiel 3:

1195 Teile feuchtes 4-Nitro-N-(2-(2-sulfato-ethylsulfonyl)-ethyl)-phthalimid werden in 2500 Teilen Wasser, 10 Teilen Natriumacetat und 8 Teilen Essigsäure gelöst. Diese Lösung wird unter Zugabe von 30 Teilen Palladium-Kohle 5% unter Normaldruck bei 50°C mit Wasserstoff bis zum Stillstand hydriert. Nach der Katalysatorfiltration wird die entstandene Lösung mit 700 Teilen Kaliumchlorid versetzt und das ausgefallene Produkt abgenutscht. Nach dem Trocknen im Vacuum bei 50°C erhält man 150 Teile 78%iges (Nitrittiter) 4-Amino-N-(2-(2-sulfato-ethylsulfonyl)-ethyl)-phthalimid der Formel 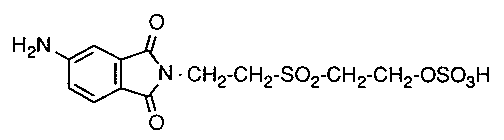
mit einer Reinheit von 96% (HPLC).
¹H-NMR-Analyse (gemessen in DMSO-d₆ mit TMS als Standard):
3,42 ppm (2H;t), 3,48 ppm (2H;t), 3,94 ppm (2H;t), 4,07 ppm (2H;t), 6,45 ppm (2H;s), 6,80 ppm (1H;q) ³J= 7 Hz und ⁴J= 2 Hz, 6,93 ppm (1H;d)⁴J= 2 Hz, 7,48 ppm (1H,d) ³J= 7 Hz.

### Beispiele 4-6a:

Analog wie in den Beispielen 1-3 beschrieben lassen sich die folgenden Verbindungen herstellen:

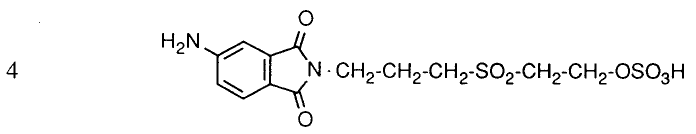

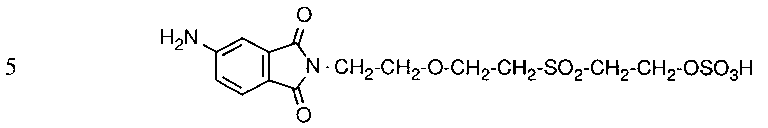

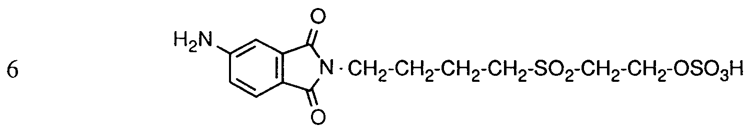

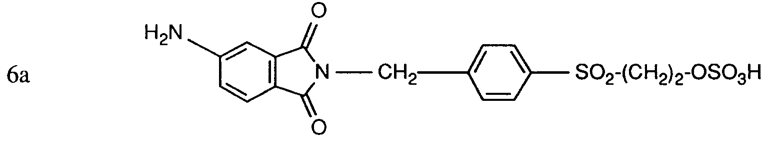

### Beispiele 7-10a:

Behandelt man die gemäss den Beispielen 3 bis 6a erhaltenen Verbindungen ca. 1 Stunde lang bei Raumtemperatur in einer wässrigen Lösung, deren pH-Wert durch Zugabe von Natriumhydroxidlösung auf ca. 10 eingestellt ist, und erniedrigt danach den pH-Wert mit Essigsäure auf 6, so erhält man ein in etwa stöchiometrisches Gemisch von zwei Verbindungen der in der Tabelle angegebenen Formel, worin sich die Aminogruppe einmal in 4-Stellung und einmal in 5-Stellung befindet.

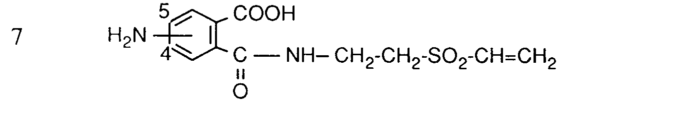

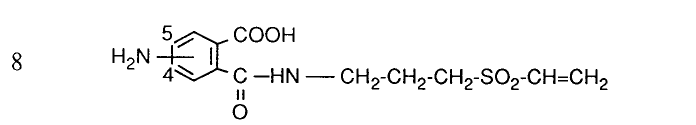

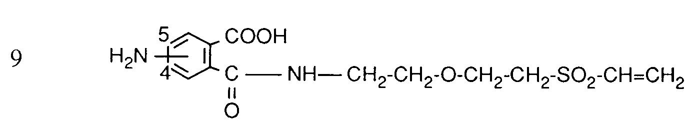

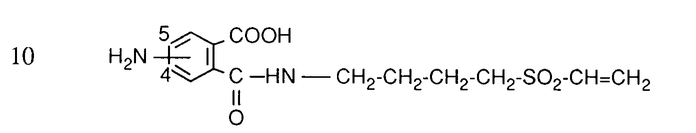

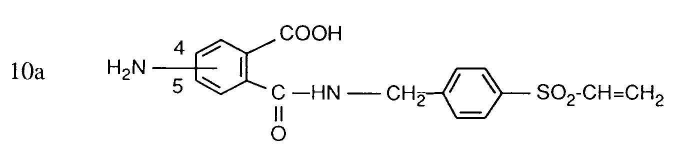

### Beispiel 11:

Zu 215 Teilen 100%iger Schwefelsäure werden bei 20 bis 30°C 32 Teile 65%iges Oleum zugetropft. Danach trägt man 37,8 Teile der Verbindung gemäss Beispiel 3 innerhalb von 30 Minuten ein und lässt dabei die Temperatur auf 60°C ansteigen. Man erwärmt die Sulfiermasse anschliessend ca. 3 Stunden auf 130°C, lässt danach auf Raumtemperatur abkühlen und trägt die abgekühlte Reaktionsmasse auf 500 Teile Eis aus. Man fällt die überschüssige Schwefelsäure mit Calciumcarbonat aus und stellt den pH-Wert des nach dem Abfiltrieren des CaSO₄ resultierenden Filtrats mit Natriumcarbonat auf einen Wert von 4 ein. Durch Eindampfen oder Gefriertrocknen erhält man die Verbindung der Formel 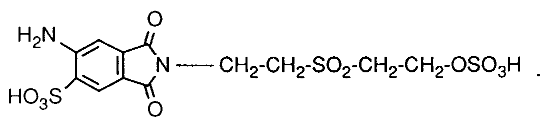
¹H-NMR-Analyse (gemessen in DMSO-d₆ mit TMS als Standard):
3,45 ppm (2H;t), 3,52 ppm (2H;t), 4,03 ppm (2H;t), 4,09 ppm (2H;t), 7,03 ppm (1H;s), 7,80 ppm (1H;s).

### Beispiel 12:

Behandelt man die gemäss Beispiel 11 erhaltene Verbindung in einem alkalischen Medium, erhält man ein in etwa stöchiometrisches Gemisch von zwei Verbindungen der Formel 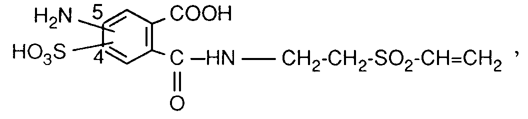
worin die eine Verbindung die Aminogruppe in 4-Stellung und die Sulfogruppe in 5-Stellung und die andere Verbindung die Aminogruppe in 5-Stellung und die Sulfogruppe in 4-Stellung aufweist.

### Beispiel 13:

37,8 Teile der Verbindung gemäss Beispiel 3 in 250 Teilen Eiswassersuspension werden mit 25 Volumenteilen 32%iger wässriger Salzsäure angesäuert und mit 25 Volumenteilen 4n Natriumnitritlösung diazotiert. Man rührt eine Stunde bei einer Temperatur von 5°C nach und zerstört dann die überschüssige salpetrige Säure mit Amidosulfonsäure. Diese Diazoniumsuspension lässt man bei 0 - 5°C und einem pH von 4,5 - 5,5 langsam in eine Suspension bestehend aus bei pH 6 in 200 Teilen Wasser angeschlämmte 1-Benzoylamino-8-hydroxynaphthalin-3,6-disulfonsäure einlaufen. Der pH wird dabei unter Zugabe von Natriumcarbonatlösung gehalten. Nach der Zugabe rührt man weitere 2 Stunden bis zur vollständigen Kupplung bei pH 5,5 nach und lässt dabei die Temperatur auf Raumtemperatur ansteigen. Dann wird die Farbstofflösung umkehrosmotisiert und gefriergetrocknet. Man erhält einen Farbstoff, der in Form der freien Säure der Verbindung der Formel 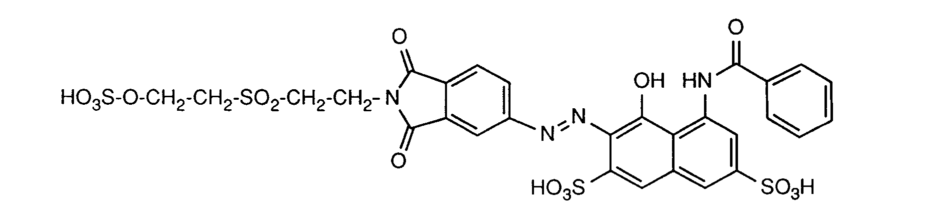
entspricht und Baumwolle und Wolle in brillanten roten Tönen mit guten Allgemeinechtheiten färbt.

### Beispiel 14:

Verfährt man wie im Beispiel 13 angegeben und stellt den pH-Wert des Reaktionsgemisches vor der Umkehrosmose mit 4n Natronlauge auf pH 10, lässt eine Stunde bei diesem pH verrühren und stellt danach den pH mit etwas Essigsäure wieder auf pH 6, so erhält man einen Farbstoff, welcher in der Form der freien Säure dem Isomerengemisch im Verhältnis 1:1 der Formel 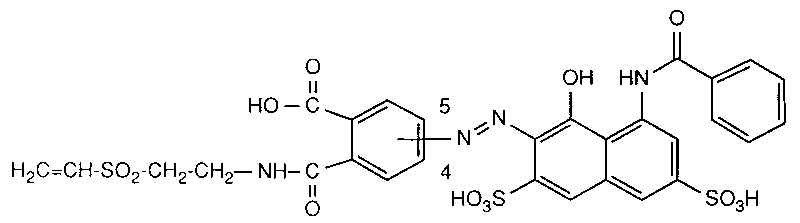
entspricht. Durch Umkehrosmose und anschliessendes Gefriertrocknen erhält man einen Farbstoff, der auf Baumwolle oder Wolle ausgefärbt die gleichen Eigenschaften wie der in Beispiel 13 angegebene Farbstoff besitzt.

## Patentansprüche

1. Verbindungen der Formel 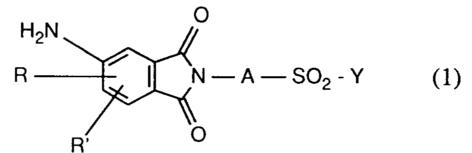 worin
R und R' unabhängig voneinander je für Wasserstoff, Sulfo, Hydroxy, C₁-C₄-Alkoxy, Halogen oder Cyano stehen,
A unsubstituiertes oder durch Halogen, Hydroxy, Sulfato, Carboxy, Cyano,
C₂-C₄-Alkanoyloxy, C₁-C₄-Alkoxycarbonyl oder Carbamoyl substituiertes oder durch eine Gruppe -O- oder -NR₁- unterbrochenes C₁-C₆-Alkylen oder unsubstituiertes oder im Phenylenteil durch C₁-C₄-Alkyl, C₁-C₄-Alkoxy, Halogen oder Sulfo substituiertes C₁-C₆-Alkylenphenylen ist,
R₁ für Wasserstoff oder C₁-C₄-Alkyl steht,
Y Vinyl oder einen Rest -CH₂-CH₂-U bedeutet, und
U eine alkalisch abspaltbare Abgangsgruppe ist.

2. Verbindungen gemäss Anspruch 1, dadurch gekennzeichnet, dass R Wasserstoff, Sulfo, Chlor, Brom oder Cyano und R' Wasserstoff bedeuten.

3. Verbindungen gemäss Anspruch 1 oder 2, dadurch gekennzeichnet, dass A einen unsubstituierten C₁-C₆-Alkylenrest oder einen Rest der Formel
-(CH₂)ₚ-O-(CH₂)_{q}-
oder
-(CH₂)ₚ- NH-(CH₂)_{q}-,
worin p und q unabhängig voneinander je eine ganze Zahl von 1 bis 6 darstellen, bedeutet.

4. Verbindungen gemäss einem der Ansprüche 1 bis 3, dadurch gekennzeichnet, dass A für C₂-C₄-Alkylen oder für einen Rest der Formel -(CH₂)₂₋₄-O-(CH₂)₂₋₄- oder -(CH₂)₂₋₄-NH-(CH₂)₂₋₄- steht.

5. Verbindungen gemäss einem der Ansprüche 1 bis 4, dadurch gekennzeichnet, dass A für 1,2-Ethylen, 1,3-Propylen, 1,4-Butylen oder einen Rest der Formel
-(CH₂)₂₋₃-O-(CH₂)₂₋₃-
oder
-(CH₂)₂₋₃- NH-(CH₂)₂₋₃-
steht.

6. Verbindungen gemäss einem der Ansprüche 1 bis 5, dadurch gekennzeichnet, dass Y Vinyl, β-Brom- oder β-Chlorethyl, β-Acetoxyethyl, β-Benzoyloxyethyl, β-Phosphatoethyl, β-Sulfatoethyl oder β-Thiosulfatoethyl und bevorzugt Vinyl oder β-Sulfatoethyl bedeutet.

7. Verbindungen der Formel (1) gemäss Anspruch 1, dadurch gekennzeichnet, dass R Wasserstoff, Sulfo, Chlor, Brom oder Cyano, R' Wasserstoff, A unsubstituiertes C₁-C₆-Alkylen oder einen Rest der Formel
-(CH₂)ₚ-O-(CH₂)_{q}-
oder
-(CH₂)ₚ-NH-(CH₂)_{q}-,
p und q unabhängig voneinander je eine ganze Zahl von 1 bis 6, und Y Vinyl, β-Brom- oder β-Chlorethyl, β-Acetoxyethyl, β-Benzoyloxyethyl, β-Phosphatoethyl, β-Sulfatoethyl oder β-Thiosulfatoethyl bedeuten.

8. Verbindungen der Formel (1) gemäss Anspruch 1, dadurch gekennzeichnet, dass R Wasserstoff oder Sulfo, R' Wasserstoff, A C₂-C₄-Alkylen oder einen Rest der Formel -(CH₂)₂₋₄-O-(CH₂)₂₋₄- oder -(CH₂)₂₋₄-NH-(CH₂)₂₋₄-, und Y Vinyl oder β-Sulfatoethyl bedeuten.

9. Verbindungen der Formel (1) gemäss Anspruch 1, dadurch gekennzeichnet, dass R und R' je Wasserstoff, A 1,2-Ethylen, 1,3-Propylen oder einen Rest der Formel -(CH₂)₂-O-(CH₂)₂- und Y β-Sulfatoethyl bedeuten.

10. Verfahren zur Herstellung von Verbindungen der Formel (1) gemäss Anspruch 1, dadurch gekennzeichnet, dass man
a) Phthalsäureanhydrid mit einer Verbindung der Formel
H₂N-A-S-CH₂-CH₂-U₁ (2)
zur Verbindung der Formel 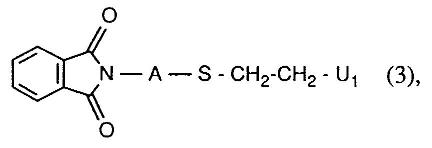 umsetzt, worin A die im Anspruch 1 angegebene Bedeutung hat und U₁ unabhängig die im Anspruch 1 für U angegebene Bedeutung hat oder für Hydroxy steht,
b) die Thioetherverbindung der Formel (3) zur entsprechenden Sulfonylverbindung der Formel 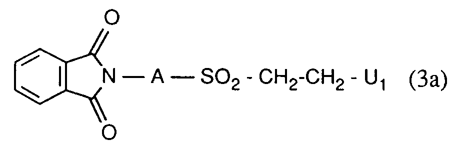 oxidiert,
c) die erhaltene Verbindung der Formel (3a) selektiv nitriert zur Verbindung der Formel 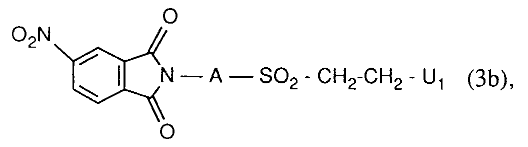
d) die erhaltene Verbindung der Formel (3b) zur Aminoverbindung der Formel 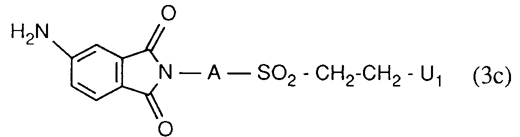 reduziert und gegebenenfalls während oder nach der Synthese der Verbindung der Formel (3a), (3b) oder (3c) einen der im Anspruch 1 genannten Reste R und/oder R' in das Molekül einführt oder den Rest U₁ in einen beliebigen Rest U umwandelt.

11. Verwendung der Verbindungen der Formel (1) gemäss Anspruch 1 als Diazokomponente zur Herstellung von faserreaktiven Mono- oder Polyazofarbstoffen.

12. Verbindungsgemisch enthaltend je eine Verbindung der Formel 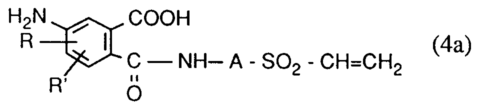 und 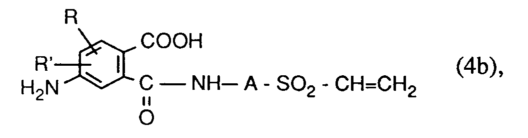 worin für A, R und R' jeweils die im Anspruch 1 angegebene Bedeutung haben.

13. Verfahren zur Herstellung eines Verbindungsgemisches enthaltend je eine Verbindung der Formel (4a) und (4b) gemäss Anspruch 12, dadurch gekennzeichnet, dass man eine Verbindung der Formel (1) gemäss Anspruch 1 in einem alkalischen Medium behandelt.

14. Verwendung eines Verbindungsgemisches enthaltend je eine Verbindung der Formel (4a) und (4b) gemäss Anspruch 12 als Diazokomponenten zur Herstellung von faserreaktiven Mono- oder Polyazofarbstoffen.
